(19) Europäisches Patentamt
European Patent Office
Office européen des brevets

(11) Veröffentlichungsnummer: **0 043 131**
**A1**

(12) # EUROPÄISCHE PATENTANMELDUNG

(21) Anmeldenummer: **81105021.0**

(22) Anmeldetag: **29.06.81**

(51) Int. Cl.³: **C 07 C 63/38**
C 07 C 51/08

(30) Priorität: **02.07.80 DE 3025016**

(43) Veröffentlichungstag der Anmeldung:
**06.01.82 Patentblatt 82/1**

(84) Benannte Vertragsstaaten:
**BE CH DE FR GB IT LI NL**

(71) Anmelder: **HOECHST AKTIENGESELLSCHAFT**
**Postfach 80 03 20**
**D-6230 Frankfurt/Main 80(DE)**

(72) Erfinder: **Frischkorn, Hans, Dr.**
**Martin-Wohmann-Strasse 3**
**D-6238 Hofheim am Taunus(DE)**

(72) Erfinder: **Schinzel, Erich, Dr.**
**Ostpreussenstrasse 43**
**D-6238 Hofheim am Taunus(DE)**

(54) **Verfahren zur Herstellung von Naphthalindicarbonsäure-(1,4).**

(57) Verfahren zur Herstellung von Naphthalin-dicarbonsäure-(1,4), dadurch gekennzeichnet, daß man 4-Bromnaphthoesäure (1) in einem polaren, aprotischen Lösungsmittel mit mindestens 1 Mol Kupfer-(I)-cyanid pro Mol 4-Bromnaphthoesäure umsetzt und den so erhaltenen Kupfersalzkomplex der 4-Cyannaphthoesäure alkalisch verseift.

EP 0 043 131 A1

# Verfahren zur Herstellung von Naphthalindicarbonsäure-(1,4)

Es ist bereits bekannt, Naphthalindicarbonsäure-(1,4) durch Verseifung des 1,4-Dicyannaphthalins herzustellen [vgl. R. Scholl und H. Neumann, Ber. 55 (1922), 121 und E.F. Bradbrook u. R.P. Linstead, J.chem.Soc. (London) 1936, 1739-1744]. Die dort beschriebenen Verfahren zur Herstellung des Dinitrils gehen aus von geeigneten Naphthalinsulfonsäuren, welche durch Kaliumcyanidschmelze in das 1,4-Dicyannaphthalin überführt werden. Diese Verfahren erfordern aber extreme Bedingungen, führen in größerem Maße zu dunkelgefärbten Nebenprodukten und sind somit für eine technische Gewinnung der Naphthalindicarbonsäure-(1,4) wenig geeignet.

Es wurde nun gefunden, daß sich Naphthalindicarbonsäure-(1,4) in hoher Reinheit und in technisch gut durchführbarer Weise herstellen läßt, wenn man 4-Bromnaphthoesäure-(1) in polaren aprotischen Lösungsmitteln mit mindestens 1 Mol Kupfer-(I)-cyanid pro Mol 4-Bromnaphthoesäure, gegebenenfalls in Gegenwart von katalytischen Mengen Kaliumjodid und Kupfersulfat umsetzt und den so erhaltenen Kupfersalzkomplex der 4-Cyannaphthoesäure alkalisch verseift.

Die nach dem erfindungsgemäßen Verfahren als Ausgangsmaterial erforderliche 4-Brom-naphthoesäure-(1) ist in der Literatur schon beschrieben worden. Sie kann z.B. in einfacher Weise aus 1-Bromnaphthalin durch Umsetzung mit Acetylchlorid nach Friedel-Crafts zum 4-Brom-1-acetylnaphthalin (vgl. K. Dziewouski u. L. Sternbach, Bull. acad. polonaise A, 1931, 59 bis 68, bzw. T.L. Jacobs, S. Winstein, J.W. Ralls u. J.H. Robson Journ. org.Chem. 11 (1946), 27-33) und dessen Oxydation mit Hypochlorit [vgl. T.L. Jacobs et. al, s.o. bzw. N.J. Leonard u. A.M.

BAD ORIGINAL

Hyson, J.Am.chem.Soc. 71 (1949), 1963] hergestellt werden.

Die angewendete Menge des Kupfer-(I)-cyanids schwankt zwischen 1 und 1,5 Mol, bezogen auf 1 Mol der 4-Brom-naphthoesäure. Als polares, aprotisches Lösungsmittel wendet man N-Methylacetamid, Dimethylacetamid, Dimethylsulfoxid, N-Methylpyrrolidon, Hexamethylphosphorsäuretriamid, Chinaldin und vorzugsweise Dimethylformamid und Chinolin an.

Die Reaktion wird bei Temperaturen von 150-250°C durchgeführt, vorteilhaft arbeitet man beim Siedepunkt des eingesetzten Lösungsmittels. Außerdem ist es vorteilhaft, die Reaktion durch Zugabe eines Katalysators zu beschleunigen. Zu diesem Zweck kann man ein Gemisch aus Kaliumjodid und Kupfersulfat nehmen.

Bei dieser Reaktion fällt die 4-Cyannaphtoesäure als Kupfer-(I)-salzkomplex folgender Zusammensetzung an

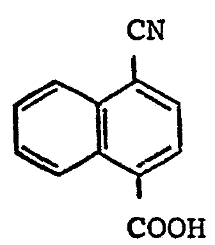 · n CuBr     n= mindestens 1

das nach Abkühlen des Reaktionsgemisches und nach Zusatz von Wasser oder eines niedrig siedenden Lösungsmittels isoliert werden kann. Als niedrig siedendes polares Lösungsmittel werden niedere Alkohole, vorzugsweise Methanol oder Ethanol verwendet.

Ohne die 4-Cyannaphthoesäure freizusetzen wird der noch feuchte Kupfersalzkomplex dann nach bekannten Methoden direkt alkalisch verseift mit einem starken Alkali, beispielsweise mit überschüssiger 10-35 %iger Natronlauge in der Siedehitze. Anschließend stellt man den pH-Wert der Lösung auf 7 bis 8 und trennt das dabei quantitativ ausgeschiedene Kupferoxid ab. Aus dem Filtrat wird durch weitere

Zugabe von Mineralsäure bis auf pH 2 die Naphthalindicarbonsäure abgeschieden, die abgetrennt wird.

Die Aufarbeitung kann auch in der Weise erfolgen, daß man auf die Isolierung des Kupfersalz-Komplexes der 4-Cyannaphthoesäure verzichtet und nach Beendigung des Halogen-Cyanid-Austausches das Reaktionsgemisch mit starkem Alkali, wie etwa 10-35 %ige Natronlauge, direkt hydrolisiert.

Verwendet man dabei Chinolin oder Chinaldin als Lösungsmittel, so kann man dies während der Hydrolyse mit Wasserdampf abdestillieren.

Die nach dem erfindungsgemäßen Verfahren erhaltene Naphthalindicarbonsäure-(1,4) findet Verwendung als Vorprodukt für Farbstoffe, UV-Absorber, Szintillatoren und optische Aufheller. Sie ist frei von gefärbten Verunreinigungen und kann direkt zur weiteren Synthese eingesetzt werden.

Die folgenden Beispiele erläutern die Erfindung näher. Temperaturen sind in °C angegeben.

Beispiel 1:

In 330 Gewichtsteilen Dimethylformamid werden 125,6 Gewichtsteile 4-Brom-naphthoesäure-(1) vom Schmelzpunkt 219 - 220° und 51 Gewichtsteile Kupfer-(I)-cyanid (67 - 71% Kupfer) eingetragen. Anschließend erhitzt man unter Rühren 4 Stunden am Rückfluß auf 150 - 160°C, wobei nach einiger Zeit Lösung eintritt, dann aber nach und nach der 4-Cyan-naphthoesäure-(1)-Kupferbromid-Komplex ausfällt. Nach Abkühlen auf ca. 100° verdünnt man das Reaktionsgemisch mit 330 Gewichtsteilen Wasser, rührt ca. eine Stunde bei Raumtemperatur nach, saugt ab und wäscht mit ca. 400 Gewichtsteilen Wasser nach. Der so isolierte 4-Cyan-naphthoesäure-Kupferbromid-Komplex wird in 550 Gewichtsteile 20 %ige Natronlauge eingetragen und 5 Stunden am Rückfluß erhitzt. Anschließend stellt man mit ca. 220 Gewichtsteilen konzentrierter Salzsäure auf pH 7,0 - 7,5, saugt vom abgeschiedenen Kupferoxidschlamm ab und wäscht diesen mit ca. 500 Gewichtsteilen Wasser portionsweise nach. Im Filtrat wird durch Zugabe von ca. 100 Gewichtsteilen konzentrierter Salzsäure die Naphthalindicarbonsäure-(1,4) bei pH 2 ausgefällt, abgesaugt und mit Wasser chlorionenfrei gewaschen. Nach Trocknen erhält man 92 Gewichtsteile einer praktisch farblosen Naphthalindicarbonsäure (1,4) mit einem Schmelzpunkt von 315 - 320°.

Die eingesetzte 4-Brom-naphthoesäure-(1) kann wie folgt hergestellt werden:

In 500 Volumenteile trockenem Ethylenchlorid werden 160 Gewichtsteile Aluminiumchlorid und anschließend 94 Gewichtsteile Acetylchlorid eingetragen. Man rührt bei 30 - 35° solange nach, bis sich eine klare Lösung gebildet hat. Erst dann gibt man bei ca. 40° 207 Gewichtsteile 1-Bromnaphthalin, in 250 Volumenteilen Ethylenchlorid gelöst, innerhalb von 15 Minuten hinzu und rührt 30 Minuten bei ca. 90° nach. Man kühlt auf Raumtemperatur, saugt den ausgefallenen Aluminiumchlorid-4-Brom-1-acetylnaphthalin-Komplex ab und wäscht diesen mehrmals mit wenig

Ethylenchlorid nach. Den erhaltenen Komplex trägt man in 2000 Volumenteile 2 normale Salzsäure ein und zersetzt diesen durch Erhitzen auf 100°, wobei man gleichzeitig miteingebrachtes Ethylenchlorid abdestilliert. Nach Abkühlen unter Rühren auf Raumtemperatur scheidet sich 4-Brom-1-acetyl-naphthalin kristallin ab. Man saugt ab, wäscht die anhaftende Säure gut mit Wasser aus, wozu man gegebenenfalls das Nutschgut mit Wasser vermahlt, und erhält nach dem Trocknen 207 Gewichtsteile eines fast farblosen Produktes vom Schmelzpunkt 46 - 48°.

Das so erhaltene 4-Brom-1-acetyl-naphthalin wird, gegebenenfalls noch feucht, in 2000 Gewichtsteile Wasser eingetragen und das Reaktionsgemisch unter Zusatz von ca.70 Gewichtsteilen konzentrierter Natronlauge und 0,1 Gewichtsteilen eines Dispergiermittels auf 90° erhitzt. Man läßt darauf innerhalb von 2 bis 3 Stunden 2000 Volumenteile Chlorbleichlauge (14 % aktives Chlor enthaltend) zufließen, wobei man entstehendes Chloroform über einen absteigenden Kühler abdestilliert. Nach einer weiteren Reaktionszeit von einer Stunde setzt man ca. 100 Volumenteile einer 40 %igen Natrium-bisulfit-Lösung zu, klärt die entstandene Lösung nach Zusatz von 5 Gewichtsteilen Aktivkohle und säuert mit ca. 80 Gewichtsteilen konzentrierter Salzsäure bis auf pH 2 an. Die ausgefallene 4-Brom-naphthoesäure-(1) wird abgesaugt, mit Wasser chlorinenfrei gewaschen und getrocknet. Man erhält 187 Gewichtsteile einer fast farblosen Säure vom Schmelzpunkt 219 - 220°.

<u>Beispiel 2:</u>

In 330 Gewichtsteile wasserfreiem Chinolin werden 125,6 Gewichtsteile 4-Brom-naphthoesäure-(1) vom Schmelzpunkt 219 - 220° und 51 Gewichtsteile Kupfer-(I)-cyanid (67 - 71 % Kupfer) eingetragen. Anschließend erhitzt man unter Rühren 4 Stunden am Rückfluß auf 235 - 240°, wobei eine homogene dunkle Lösung entsteht. Nach Abkühlen auf ca.

/

120° versetzt man das Reaktionsgemisch mit 750 Gewichtsteilen einer 15 %igen Natronlauge und erhitzt die erstandene gelblichbraune Suspension am Rückfluß, wobei der Rücklauf über ein geeignetes Phasentrenngerät geleitet wird und man die spezifisch schwerere Phase - wasserhaltiges Chinolin - abzieht. Nach vollständiger Verseifung des 4-Cyan-naphthoesäure-(1)-Kupfersalz-Komplexes bzw. vollständiger Abtreibung des Chinolins (nach ca. 6 Stunden) läßt man auf 80° abkühlen, stumpft mit ca. 230 Gewichtsteilen konzentrierter Salzsäure auf pH 7,0 - 7,5 ab, saugt von abgeschiedenen Kupferoxidschlamm ab und wäscht diesen mit ca. 500 Gewichtsteilen Wasser nach. Im Filtrat fällt man die Naphthalindicarbonsäure-(1,4) durch Zugabe von ca. 90 Gewichtsteilen konzentrierter Salzsäure bei pH 2 aus, saugt ab und wäscht mit Wasser chlorionenfrei. Nach Trocknen erhält man 90 Gewichtsteile einer praktisch farblosen Säure mit einem Schmelzpunkt von 315 - 320°.

Patentansprüche:

1. Verfahren zur Herstellung von Naphthalin-dicarbonsäure-(1,4), dadurch gekennzeichnet, daß man 4-Bromnaphthoesäure(1) in einem polaren, aprotischen Lösungsmittel mit mindestens 1 Mol Kupfer-(I)-cyanid pro Mol 4-Bromnaphthoesäure umsetzt und den so erhaltenen Kupfersalzkomplex der 4-Cyannaphthoesäure alkalisch verseift.

2. Verfahren nach Anspruch 1, dadurch gekennzeichnet, daß man den Kupfersalzkomplex der 4-Cyannaphthoesäure durch Zugabe von Wasser oder einem niedrig siedenden polaren Lösungsmittel ausfällt, abtrennt und verseift.

3. Verfahren nach Anspruch 1, dadurch gekennzeichnet, daß man den Kupfersalzkomplex der 4-Cyannaphthoesäure nach dem Cyanidaustausch direkt ohne vorherige Isolierung in dem Reaktionsgemisch hydrolisiert.

Europäisches Patentamt

# EUROPÄISCHER RECHERCHENBERICHT

Nummer der Anmeldung

EP 81 10 5021.0

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | JOURNAL OF ORGANIC CHEMISTRY, Band 41, Nr. 6, 1976 Columbus J.M. McNAMARA et al. "Synthesis of 4-Cyano-4'-halobiphenyls" Seite 1071 -- | 1 | C 07 C 63/38<br>C 07 C 51/08 |
| A | DE - A - 2 249 880 (THE LUMMUS CO.) * Ansprüche 1, 7 * -- | 1 | |
| A | DE - A - 2 417 362 (SUN VENTURES) * Anspruch 1 * -- | 1 | RECHERCHIERTE SACHGEBIETE (Int. Cl.³)<br>C 07 C 51/00<br>C 07 C 51/08<br>C 07 C 63/38<br>C 07 C 121/62 |
| | CHEMISCHES ZENTRALLBLATT, Band 90, Nr. 25, 1919 III, Berlin K.W.ROSENMUND et al. "Das am Ringkohlenstoff gebundene Halogen und sein Ersatz durch andere Substituenten. 1. Mitteilung: Ersatz des Halogens durch die Carboxylgruppe" Seite 1055 -- | 1 | |
| A | CHEMISCHES ZENTRALLBLATT, Band 109, Nr. 3, 1938 I, Berlin M.S. NEWMAN "Die Darstellung von 1-Naphthonitril" Seiten 587 bis 588 & J. Amer. chem. Soc. Band 59, 1937 Seite 2472 ---- | 1 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |

X Der vorliegende Recherchenbericht wurde für alle Patentansprüche erstellt.

| Recherchenort | Abschlußdatum der Recherche | Prüfer |
|---|---|---|
| Berlin | 02-10-1981 | KNAACK |